# EUROPEAN PATENT SPECIFICATION

(11) **EP 3 450 116 B1**
(45) Date of publication and mention of the grant of the patent: **29.03.2023**
(21) Application number: 18186676.5
(22) Date of filing: 31.07.2018
(51) Int. Cl.: B25J 5/02, B05C 11/10, B25J 11/00, B29B 13/02, A61F 13/15, B29C 65/48

(54) **APPARATUS AND METHOD FOR MANUFACTURING ABSORBENT ARTICLE**
VORRICHTUNG UND VERFAHREN ZUR HERSTELLUNG EINES SAUGFÄHIGEN ARTIKELS
APPAREIL ET PROCÉDÉ DE FABRICATION D'UN ARTICLE ABSORBANT

(30) Priority: 30.08.2017 JP 2017165583
(43) Date of publication of application: 06.03.2019
(73) Proprietor: Unicharm Corporation, Shikokuchuo-shi, Ehime 799-0111 (JP)
(72) Inventor: HAGITA, Hiromi, Kanonji-shi, Kagawa 769-1602 (JP); TASHIRO, Fumihiro, Kanonji-shi, Kagawa 769-1602 (JP)
(74) Representative: Dolleymores

(56) References cited:
- DE-A1- 3 310 698
- FR-A1- 2 579 114
- JP-B1- 6 089 158

## Description

### BACKGROUND

The present disclosure relates to an apparatus and method for manufacturing an absorbent article.

### Description of the Related Art

A common absorbent article is manufactured such that a plurality of materials is laminated and joined to adhere to each other with a hot-melt adhesive. Thus, an absorbent article manufacturing apparatus includes a melting tank to store a melted hot-melt adhesive in a liquid state.

Conventionally, the melting tank storing the hot-melt adhesive having been reduced by being used for joining is manually refilled. Thus, there is a case where the melting tank is not refilled until immediately before running out of the hot-melt adhesive. In such a case, a worker charges a large amount of hot-melt adhesive, at a time, into the melting tank where the hot-melt adhesive hardly exists. Then, when such a situation occurs, there is a problem that the hot-melt adhesive deteriorates in quality (Japanese Patent Application Publication No. H11-28410)
FR 2579114 A1 relates to an improved installation for dispensing hot-melt adhesive, including a probe sensitive to variations in the level of molten adhesive.
JP 6089158 B1 relates to an absorbent article manufacturing apparatus including a hot-melt apparatus.

The present disclosure has been made in view of such an issue as described above, and an aspect thereof is to prevent deterioration in quality of a hot-melt adhesive.

### SUMMARY

The present invention provides the apparatus for manufacturing an absorbent article by bonding a material with a hot-melt adhesive of independent claim 1 and the method for manufacturing an absorbent article by bonding a material with a hot-melt adhesive of independent claim 10. The dependent claims specify preferred but optional features.

A primary aspect of the disclosure is an apparatus for manufacturing an absorbent article by bonding a material with a hot-melt adhesive, the apparatus comprising: a melting tank configured to melt the hot-melt adhesive; a detection sensor configured to detect an amount of the hot-melt adhesive in the melting tank; and an articulated robot which is a device configured to hold the hot-melt adhesive in a container containing the hot-melt adhesive and to move the hot-melt adhesive from the container containing the hot-melt adhesive to the melting tank based on a detection result of the detection sensor.

Other features of the present invention will become apparent from descriptions of the present specification and of the accompanying drawings.

### BRIEF DESCRIPTION OF THE DRAWINGS

FIG. 1 is a top-face conceptual diagram illustrating an absorbent article manufacturing apparatus 10 according to an embodiment of the present disclosure (first embodiment) when viewed from above.
FIG. 2 is a perspective conceptual diagram illustrating an absorbent article manufacturing apparatus 10 according to an embodiment of the present disclosure (first embodiment) when viewed obliquely.
FIG. 3 is a flow chart illustrating operational control of a robot 20.
FIG. 4 is a flow chart illustrating operational control of an AGV40.

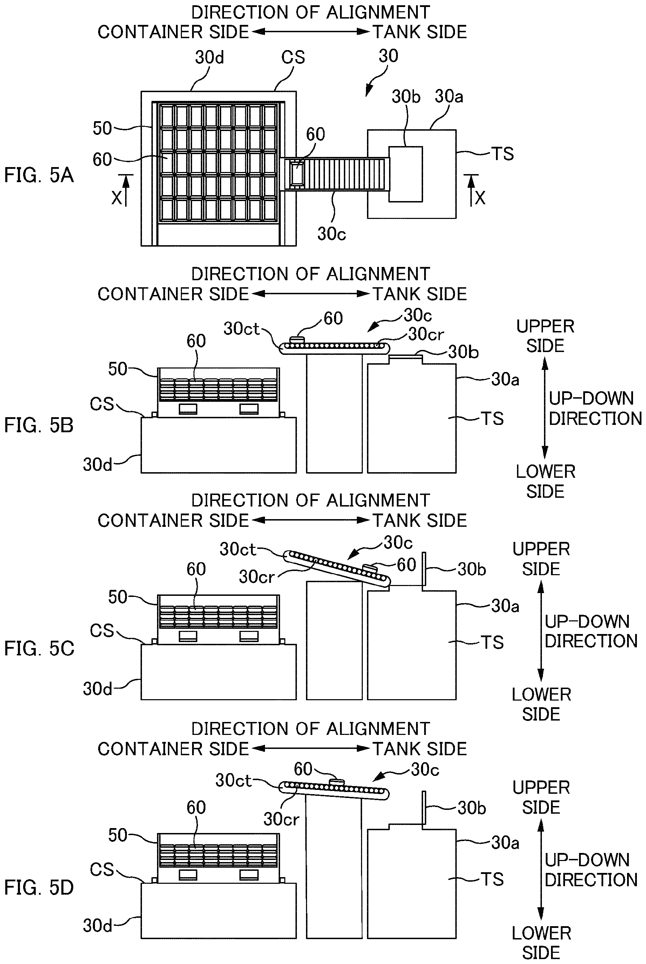

FIG. 5A is a top-face conceptual diagram illustrating a hot-melt device 30 when viewed from above. FIG. 5B is a view (side view) along arrows X-X of FIG. 5A and a side-surface conceptual diagram illustrating a position (movement waiting position) at which an intermediate charge portion 30c is waiting for a hot-melt adhesive 60 to be moved by a robot 20. FIG. 5C is a side-face conceptual diagram illustrating a position (charging position) at which an intermediate charge portion 30c charges a hot-melt adhesive 60 into a melting tank 30a. FIG. 5D is a side-face conceptual diagram illustrating an intermediate charge portion 30c according to another embodiment (second embodiment) .
FIG. 6 is a top-face conceptual diagram illustrating an absorbent article manufacturing apparatus 10 according to a third embodiment when viewed from above.

### DETAILED DESCRIPTION

At least following matter will become clear from the descriptions of the present specification with reference to the accompanying drawings.

An apparatus for manufacturing an absorbent article by bonding a material with a hot-melt adhesive, the apparatus comprising: a melting tank configured to melt the hot-melt adhesive; a detection sensor configured to detect an amount of the hot-melt adhesive in the melting tank; and an articulated robot which is a device configured to hold the hot-melt adhesive in a container containing the hot-melt adhesive and to move the hot-melt adhesive from the container containing the hot-melt adhesive to the melting tank based on a detection result of the detection sensor.

According to such an absorbent article manufacturing apparatus, it becomes possible to restrain deterioration in quality of the hot-melt adhesive.

It is preferable that such an absorbent article manufacturing apparatus further comprises: a plurality of melting tanks, wherein the robot in common is configured to move the hot-melt adhesive to each of the plurality of melting tanks.

According to such an absorbent article manufacturing apparatus, it becomes possible to reduce the number of robots as compared with an example in which robots are respectively dedicated to a plurality of melting tanks.

In such an absorbent article manufacturing apparatus, it is preferable that the robot is a self-propelled robot, and when the robot is self-propelled to reach a corresponding position, the corresponding position corresponding to one melting tank among the plurality of melting tanks, the robot moves the hot-melt adhesive to the one melting tank.

According to such an absorbent article, the number of robots can be further reduced.

It is preferable that such an absorbent article manufacturing apparatus further comprises: a container transport member configured to be self-propelled to transport the container, wherein the container transport member is configured to install the transporting container in the container installation position, and to transport the used container collected from the container installation position.

According to such an absorbent article manufacturing apparatus, it becomes possible to perform work of replacing containers swiftly.

It is preferable that such an absorbent article manufacturing apparatus further comprises: a self-propelled robot lane along which the robot is self-propelled; a transport member running lane along which the container transport member is self-propelled; and a tank container installation zone in which the plurality of melting tanks and containers are aligned in the direction of alignment, wherein the self-propelled robot lane and the transport member running lane are located on both sides, in a direction orthogonal to a direction of alignment, of the tank container installation zone, and the self-propelled robot lane and the transport member running lane are both along the direction of alignment.

According to such an absorbent article manufacturing apparatus, it becomes possible to implement efficient movements of the robot and the container transport member.

In such an absorbent article, it is preferable that the container transport member is configured to transport a plurality of containers containing hot-melt adhesives different from one another, and the plurality of containers has same specifications.

According to such an absorbent article manufacturing apparatus, it becomes possible to negate the need to prepare a plurality of container transport members corresponding to the specifications of the containers, and reduce the costs related to such container transport members.

It is preferable that such an absorbent article manufacturing apparatus further comprises: a residual detecting portion configured to detect that no residual hot-melt adhesive adheres to the robot, when the robot completes movement of the hot-melt adhesive.

According to such an absorbent article manufacturing apparatus, when a hot-melt adhesive of a type different from the type of the residual hot-melt adhesive is held by the robot, such hot-melt adhesives can be restrained from being mixed together.

It is preferable that such an absorbent article manufacturing apparatus further comprises: an intermediate charge portion configured to receive the hot-melt adhesive from the robot, and to charge the received hot-melt adhesive to the melting tank, the intermediate charge portion being provided adjacent to the melting tank, wherein the robot is configured to move the hot-melt adhesive to the melting tank via the intermediate charge portion.

According to such an absorbent article manufacturing apparatus, since the robot does not move above the opening portion of the melting tank, it is possible to prevent adverse effects of oil smoke onto the robot.

In such an absorbent article, it is preferable that the melting tank includes an openable/closable lid portion, and when the intermediate charge portion charges the hot-melt adhesive into the melting tank, the lid portion is opened.

According to such an absorbent article manufacturing apparatus, it becomes possible to restrain oil smoke from being discharged from the opening portion.

A method for manufacturing an absorbent article by bonding a material with a hot-melt adhesive, the method comprising: detecting, using a detection sensor, an amount of the hot-melt adhesive in a melting tank, the melting tank configured to melt the hot-melt adhesive; holding the hot-melt adhesive, using an articulated robot, in a container containing hot-melt adhesive and moving the hot-melt adhesive, using the articulated robot, from the container containing the hot-melt adhesive to the melting tank based on a detection result of the detection sensor.

According to such a method for manufacturing an absorbent article, the same function and effects can be exerted as those in the case of the aforementioned absorbent article manufacturing apparatus.

### ===Absorbent article manufacturing apparatus 10 according to an embodiment of the present disclosure===

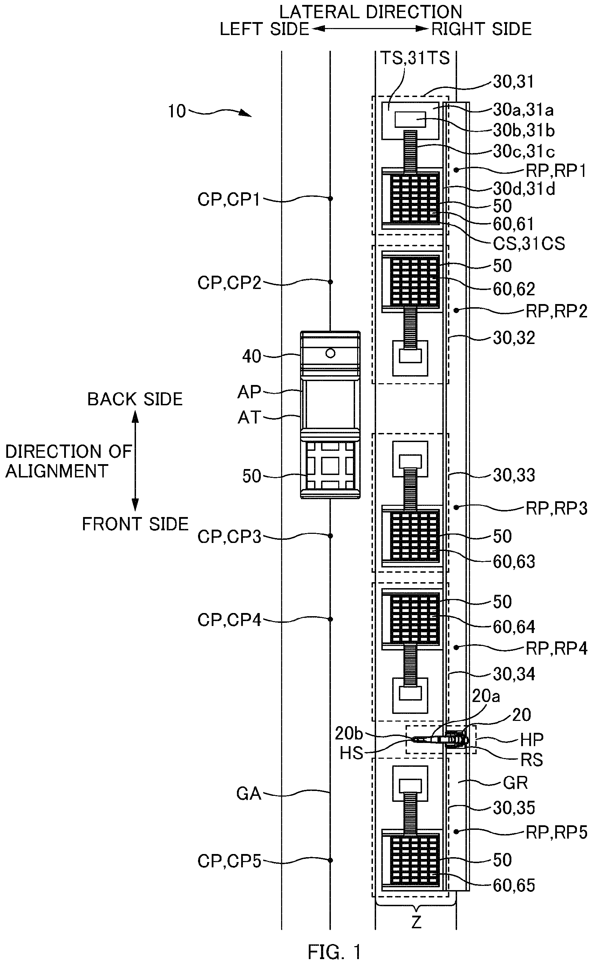

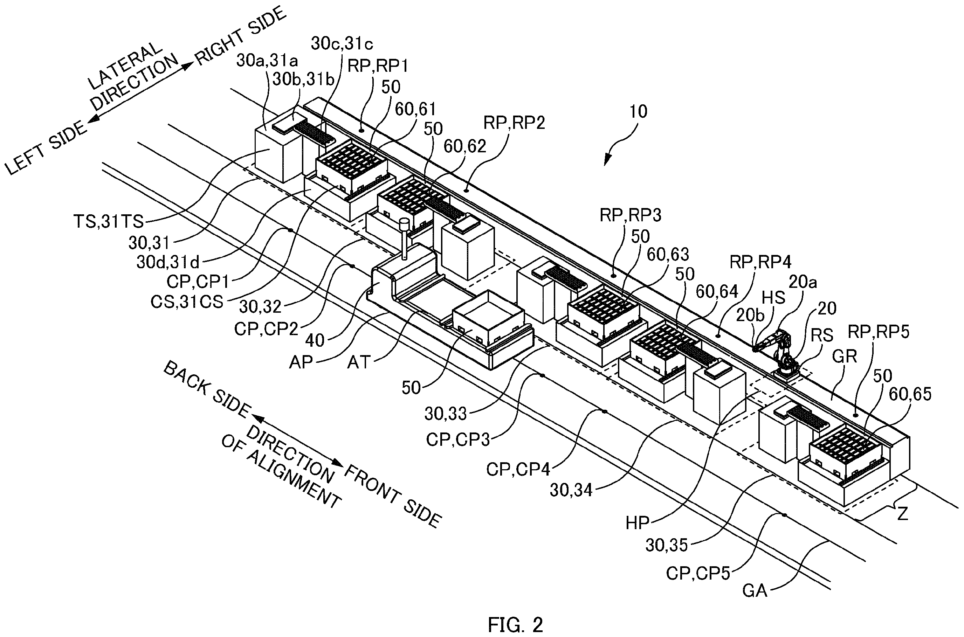

FIG. 1 is a top-face conceptual diagram illustrating an absorbent article manufacturing apparatus 10 according to an embodiment of the present disclosure when viewed from above. FIG. 2 is a perspective conceptual view illustrating the absorbent article manufacturing apparatus 10 according to an embodiment of the present disclosure when viewed obliquely. The absorbent article manufacturing apparatus 10 (absorbent article manufacturing method) is an apparatus (method) for manufacturing an absorbent article by bonding a material using a hot-melt adhesive. Note that the absorbent article includes, for example, open-type and pull-on type disposable diapers, a sanitary napkin, a urine absorbing pad, and the like, however, any absorbent article may be employed as long as it absorbs a wearer's excreted fluid. Further, a continuous sheet of fiber assembly, for example, a soft and flexible continuous sheet such as a nonwoven fabric or a tissue is used as a material.

The absorbent article manufacturing apparatus 10 according to an embodiment of the present disclosure includes, as illustrated in FIGS. 1 and 2, a robot 20, a residual detection portion HS, a plurality of hot-melt devices 30, an AGV 40 (automated guided vehicle: corresponding to a container transport member), a self-propelled robot lane GR along which the robot 20 is self-propelled, and a transport member running lane GA along which the AGV 40 is self-propelled. The plurality of hot-melt devices 30 each includes a melting tank 30a, a melting tank lid portion 30b (corresponding to a lid portion), an intermediate charge portion 30c, a container installation portion 30d, an in-tank detection sensor TS (corresponding to a detection portion to detect the amount of a hot-melt adhesive 60 in the melting tank 30a), and a detection portion (hereinafter, referred to as an in-container detection portion CS) to detect the amount of the hot-melt adhesive 60 in a container 50. Note that the absorbent article manufacturing apparatus 10 includes a device to process a material (not shown), a device to feed a material (not shown), and/or the like, in addition to devices relating to adhering described above, however, the detailed descriptions thereof are omitted.

Further, in an embodiment of the present disclosure, a direction (horizontal direction) in which the plurality of hot-melt devices 30 is aligned is given as a direction of alignment, a direction (horizontal direction) orthogonal to the direction of alignment on the paper of FIG. 1 is given as a lateral direction (horizontal direction), a direction penetrating the paper of FIG. 1 is given as an up-down direction (vertical direction). Further, the back side (front side) in the direction of alignment in the paper is referred to as a "back side (front side)", and the left side (right side) in the lateral direction in the paper is referred to as a "left side (right side)".

### <<<Robot 20>>>

The robot 20 is a device to hold the hot-melt adhesive 60 in the container 50 provided in a tank container installation zone Z, which will be described later, and move it to the melting tank 30a (may also be said as a device to move it to the intermediate charge portion 30c).

This robot 20 is a so-called articulated robot, and includes an arm 20a provided with a plurality of articulations. The arm 20a includes a holding portion 20b to hold the hot-melt adhesive 60. An embodiment of the present disclosure uses a plurality of types of the hot-melt adhesive 60 and, for example, five types of the hot-melt adhesive 60 in FIGS. 1 and 2. Then, the holding portion 20b of the robot 20 according to an embodiment of the present disclosure can hold the five types of the hot-melt adhesive 60.

Further, in an embodiment of the present disclosure, provided is the residual detection portion HS to detect that no residual hot-melt adhesive 60 adheres to the robot 20 when the robot 20 completes the movement of the hot-melt adhesive 60. That is, every time when the hot-melt adhesive 60 is moved, the residual detection portion HS detects whether the moved hot-melt adhesive 60 remains in the holding portion 20b of the robot 20. Then, in an embodiment of the present disclosure, the residual detection portion HS is provided to the robot 20, the residual detection portion HS may be, for example, the residual detection portion HS using an imaging device to image the holding portion 20b, and/or a pressure detection portion to detect change in pressure of the holding portion 20b.

Further, the robot 20 according to an embodiment of the present disclosure is a so-called self-propelled robot capable of being self-propelled along the direction of alignment as a direction of movement. In other words, the absorbent article manufacturing apparatus 10 includes the self-propelled robot lane GR along which the robot 20 is self-propelled, and the self-propelled robot lane GR is provided, along the direction of alignment, at a position adjacent, in the lateral direction, to the tank container installation zone Z (on the right side of the tank container installation zone Z). The robot 20 includes a drive portion for self-propelling (not shown) and a robot position detection portion RS to detect a current position. The robot position detection portion RS includes, for example, those using a magnetic sensor or a laser sensor. Further, corresponding positions RP for moving the five types of the hot-melt adhesive 60 are provided in the self-propelled robot lane GR. In FIGS. 1 and 2, five corresponding positions RP (for convenience sake, referred to as a first corresponding position RP1, a second corresponding position RP2, a third corresponding position RP3, a fourth corresponding position RP4, and a fifth corresponding position RP5 in the order from the back side to the front side) are provided corresponding to the five hot-melt devices 30 (melting tanks 30a) which will be described later. The robot position detection portion RS detects the corresponding positions RP, so that the robot 20 can be self-propelled to positions to which the five types of the hot-melt adhesive 60 are moved, respectively. That is, in an embodiment of the present disclosure, the robot 20 is a self-propelled robot, and when the robot 20 is self-propelled to reach the corresponding position RP which is one of the plurality of melting tanks 30a, the robot 20 moves the hot-melt adhesive 60 to the one melting tank 30a.

Further, the number of the robots 20 is equal to or smaller than the number of types of the hot-melt adhesive 60. In an embodiment of the present disclosure, the single robot 20 is provided for the five types of the hot-melt adhesive 60, as illustrated in FIGS. 1 and 2.

In an embodiment of the present disclosure, as described above, the five types of the hot-melt devices 30 are used, and five hot-melt devices 30 are provided to five types of the hot-melt adhesive 60, respectively. FIGS. 1 and 2 illustrate the five hot-melt devices 30 corresponding to the five types of the hot-melt adhesive 60. These hot-melt devices 30 are referred to as a first hot-melt device 31, a second hot-melt device 32, a third hot-melt device 33, a fourth hot-melt device 34, and a fifth hot-melt device 35 in the order in the direction from the back side to the front side. Then, these hot-melt devices 30 are provided in the tank container installation zone Z along the direction of alignment.

Further, in an embodiment of the present disclosure, the five hot-melt devices 30 (the first hot-melt device 31 to the fifth hot-melt device 35) are provided as the hot-melt devices 30. Thus, the five types of the hot-melt adhesive 60 (for convenience sake, referred to as a first hot-melt adhesive 61, a second hot-melt adhesive 62, a third hot-melt adhesive 63, a fourth hot-melt adhesive 64, and a fifth hot-melt adhesive 65), which are respectively used for the first hot-melt device 31 to the fifth hot-melt device 35, are respectively provided, in a state of being housed in the containers 50, in the container installation portions 30d (corresponding to container installation portions) of the tank container installation zone Z.

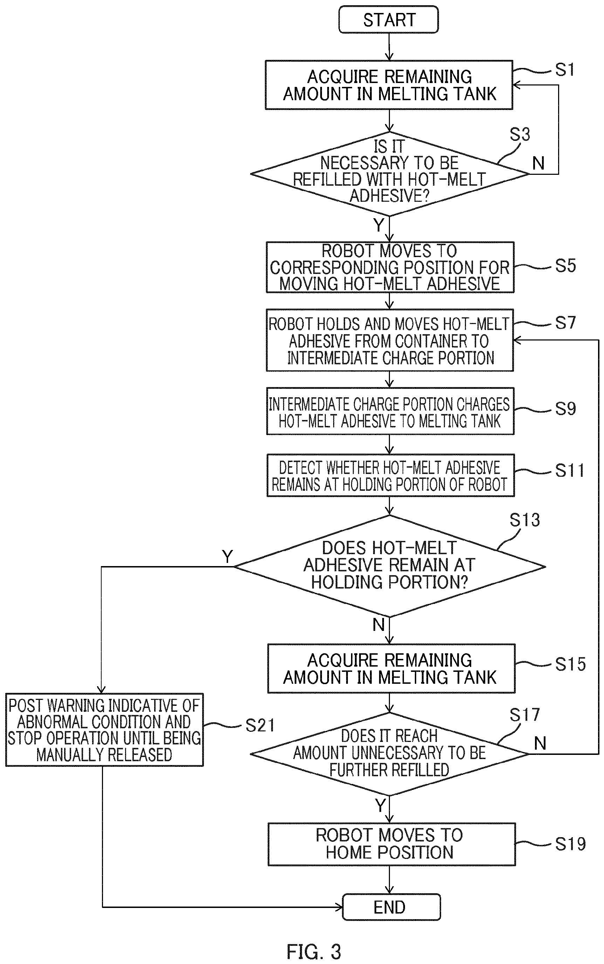

FIG. 3 is a flow chart illustrating operational control of the robot 20. An operation of the robot 20 according to an embodiment of the present disclosure will be described with reference to the flow chart. Here, a description will be made by providing an example of a movement of the first hot-melt adhesive 61 with respect to the first hot-melt device 31 out of the five hot-melt devices 30. For convenience sake, the portions included in the first hot-melt device 31 are referred to as a first melting tank 31a, a first melting tank lid portion 31b, a first intermediate charge portion 31c, a first container installation portion 31d, a first in-tank detection sensor 31TS, and a first in-container detection portion 31CS.

First, the amount of the first hot-melt adhesive 61 in the first melting tank 31a is acquired by the first in-tank detection sensor 31TS (corresponding to a detection process: step S1). Then, when the amount of the first hot-melt adhesive 61 is greater than the amount set as being necessary to refill (hereinafter, referred to as a first threshold value), a state of waiting for next acquisition is brought about (No in step S3) . When the amount is smaller than the first threshold value (when it is necessary to refill the first melting tank 31a with the first hot-melt adhesive 61), the next operation is performed (Yes in step S3). That is, the robot 20 according to an embodiment of the present disclosure performs the next operation based on the detection result of the first in-tank detection sensor 31TS.

Next, the robot 20 is self-propelled to the first corresponding position RP1 of the self-propelled robot lane GR (step S5). That is, the robot 20 detects the current position using the robot position detection portion RS, to acquire a route for self-propelling on the self-propelled robot lane GR, from the difference between the current position and the first corresponding position RP1, and is self-propelled along the route to the position at which the first corresponding position RP1 is detected by the robot position detection portion RS.

Subsequently, the robot 20 acquires the first hot-melt adhesive 61 installed in the first container installation portion 31d. That is, the robot 20 extends the arm 20a to hold the first hot-melt adhesive 61 with the holding portion 20b.

Subsequently, the robot 20 moves the held first hot-melt adhesive 61 to the first intermediate charge portion 31c provided at a position adjacent to the first melting tank 31a in the tank container installation zone Z (corresponding to a movement process: step S7). That is, the robot 20 moves the arm 20a above the first intermediate charge portion 31c and releases holding of the holding portion 20b. Below the holding portion 20b, the first intermediate charge portion 31c waits at a position for receiving the first hot-melt adhesive 61 (movement waiting position which will be described later), and the first hot-melt adhesive 61 drops downward from the holding portion 20b, thereby moving to the first intermediate charge portion 31c. That is, the robot 20 moves the first hot-melt adhesive 61 from the container 50 that contains the first hot-melt adhesive 61 to the first melting tank 31a (to the first intermediate charge portion 31c).

Subsequently, the first intermediate charge portion 31c charges the first hot-melt adhesive 61 moved by the robot 20 into the first melting tank 31a (step S9). The charging operation of the first intermediate charge portion 31c will be described in the section of the hot-melt devices 30 described later.

Subsequently, the robot 20 detects whether the first hot-melt adhesive 61 remains in the holding portion 20b using the residual detection portion HS (step S11). Then, when the residual detection portion HS detects the residual of the first hot-melt adhesive 61 (Yes in step S13), the robot 20 issues (posts) a warning to notify an abnormal condition, and stops its operation until being manually released (step S21). When there is no residue, the next operation is performed (No in step S13).

Subsequently, the robot 20 acquires the amount of the first hot-melt adhesive 61 in the first melting tank 31a from the first in-tank detection sensor 31TS, to perform an operation in accordance with the acquisition result. That is, the first in-tank detection sensor 31TS detects whether the amount of the first hot-melt adhesive 61 in the first melting tank 31a reaches the amount set as being unnecessary to further refill the tank (hereinafter, referred to as a second threshold value) by virtue of the charge of the first hot-melt adhesive 61 into the first melting tank 31a described above (step S15). Then, when the detected amount does not reach the second threshold value (No in step S17), the robot 20 continues moving the first hot-melt adhesive 61. When the detected amount reaches the second threshold value (Yes in step S17), the robot 20 stops moving the first hot-melt adhesive 61, to perform the next operation. That is, the robot 20 according to an embodiment of the present disclosure performs the next operation based on the detection result of the first in-tank detection sensor 31TS.

Subsequently, the robot 20 moves to a home position HP illustrated in FIGS. 1 and 2, to wait until the next operation (step S19).

### «<Hot-melt device 30»>

The hot-melt device 30 is a device that melts a pre-melting adhesive (i.e., the hot-melt adhesive 60 in a solid state), to produce the hot-melt adhesive 60 to be applied to a material processed in a processing section. In an embodiment of the present disclosure, as described above, the five hot-melt devices 30 are provided corresponding to the five types of the hot-melt adhesive 60.

The hot-melt devices 30 include, as described above, the melting tank 30a, the melting tank lid portion 30b, the intermediate charge portion 30c, the container installation portion 30d, the in-tank detection sensor TS (detection portion to detect the amount of the hot-melt adhesive 60 in the melting tank 30a), and the in-container detection portion CS (detection portion to detect the amount of the hot-melt adhesive 60 in the container 50) .

As illustrated in FIGS. 1 and 2, in the hot-melt devices 30, the melting tank 30a, the intermediate charge portion 30c, and the container installation portion 30d are provided, in positions adjacent to each other in the direction of alignment, in the tank container installation zone Z. Then, as illustrated in FIGS. 1 and 2, the order of the melting tank 30a, the intermediate charge portion 30c, and the container installation portion 30d in the direction of alignment may be either the order of the melting tank 30a, the intermediate charge portion 30c, and the container installation portion 30d, or the order of the container installation portion 30d, the intermediate charge portion 30c, and the melting tank 30a.

The melting tank 30a according to an embodiment of the present disclosure is a tank to melt the hot-melt adhesive 60 in a solid state, and store the hot-melt adhesive 60 melted into a liquid state, as well as supply the hot-melt adhesive 60 in a liquid state to the processing section. The melting tank 30a according to an embodiment of the present disclosure includes a heating section to heat the hot-melt adhesive 60 turned into a liquid state in the melting tank 30a, a temperature detecting section to detect a temperature of the hot-melt adhesive 60 in a liquid state, and an adhesive supply section to supply the hot-melt adhesive 60 turned into a liquid state in the melting tank 30a to the processing section.

The hot-melt adhesive 60 turned into a liquid state in the melting tank 30a according to an embodiment of the present disclosure is controlled so as to be made within a set range or at a constant temperature. That is, the temperature of the hot-melt adhesive 60 in a liquid state is detected by the aforementioned temperature detecting section. When the detected temperature is lower than the set temperature, the hot-melt adhesive 60 is heated by the aforementioned heating section. When the detected temperature is higher than the set temperature, and when the hot-melt adhesive 60 is being heated by the aforementioned heating section, heating is stopped, and when the hot-melt adhesive 60 is not being heated by the aforementioned heating section, the next detection is awaited as it is. Further, the adhesive supply section according to an embodiment of the present invention reaches the processing section through a passage tube (not shown), and discharges the melted hot-melt adhesive 60 in a liquid state from a nozzle at the tip thereof toward a material, thereby supplying the hot-melt adhesive 60 to the processing section.

The in-tank detection sensor TS according to an embodiment of the present disclosure detects the amount of the hot-melt adhesive 60 in the melting tank 30a. Then, the robot 20 operates based on the detection result. In an embodiment of the present disclosure, the in-tank detection sensor TS is provided in the melting tank 30a, and this in-tank detection sensor TS includes, for example, those using a capacitance sensor and an ultrasonic sensor. Then, the amount of the hot-melt adhesive 60 turned into a liquid state in the melting tank 30a according to an embodiment of the present disclosure is controlled so as to be made within the predetermined range. Here, the amount in the predetermined range indicates the amount in a range where excessive heating of the hot-melt adhesive 60, which will be described later, is prevented, as well as the amount in a range where the amount of the hot-melt adhesive 60 turned into a liquid state in the melting tank 30a is not too much but is appropriate. The amount within the predetermined range is set in each of the melting tanks 30a. In an embodiment of the present disclosure, the amount of the hot-melt adhesive 60 in the melting tank 30a is detected by the in-tank detection sensor TS. When the detected amount is smaller than the amount as being necessary to be refilled (first threshold value), the robot 20 moves the hot-melt adhesive 60 in a solid state until the hot-melt adhesive 60 reaches the amount set as being unnecessary to be further refilled (second threshold value). Under such control of movement of the hot-melt adhesive 60, the amount of the hot-melt adhesive 60 turned into a liquid state in the melting tank 30a is controlled within the predetermined range.

The melting tank lid portion 30b according to an embodiment of the present disclosure is a lid to open/close an opening portion positioned above the melting tank 30a in the height direction. Then, this opening portion is provided in order to charge (refill) the melting tank 30a with the hot-melt adhesive 60. Further, the melting tank lid portion 30b opens/closes the opening portion in conjunction with the intermediate charge portion 30c. That is, in an embodiment of the present disclosure, the melting tank 30a includes the melting tank lid portion 30b that is openable/closable, and when the intermediate charge portion 30c charges the hot-melt adhesive 60 into the melting tank 30a, the melting tank lid portion 30b is opened. The operation of the melting tank lid portion 30b will be described later.

The intermediate charge portion 30c according to an embodiment of the present disclosure receives the hot-melt adhesive 60 from the robot 20, the received hot-melt adhesive 60 is charged into the melting tank 30a. The intermediate charge portion 30c according to an embodiment of the present disclosure includes: a substantially rectangular table 30ct that is elongated in the direction of alignment and has a flat shape on the top side thereof, to receive the hot-melt adhesive 60 from the robot 20 and transport it above the opening portion of the melting tank 30a; and an actuator (not shown) to move the table 30ct from a movement waiting position to a charging position. Then, in an embodiment of the present disclosure, as illustrated in FIGS. 5B and 5C, the table 30ct includes a plurality of transport rolls 30cr to transport the received hot-melt adhesive 60 in the direction of alignment of the table 30ct. The plurality of transport rolls 30cr form a top surface of the table 30ct (that is, the plurality of transport rolls 30cr are positioned at the upper end of the table 30ct as well as are provided from one end to the other end in the direction of alignment), and rotate about rotation shafts along the lateral direction (in the direction of penetrating the paper in FIG. 5B and FIG. 5C) .

FIGS. 5A to 5C are diagrams illustrating the operation in which the intermediate charge portion 30c having received the hot-melt adhesive 60 from the robot 20 charges the hot-melt adhesive 60 into the melting tank 30a. In FIGS. 5A to 5D, the side on which the melting tank 30a is provided, in the direction of alignment, when viewed from the intermediate charge portion 30c is referred to as a tank side, while the side on which the container installation portion 30d is provided, in the direction of alignment, when viewed from the intermediate charge portion 30c is referred to as a container side.

FIG. 5A is a top-face conceptual diagram illustrating the hot-melt devices 30 when viewed from above, FIG. 5B is a view along arrows X-X (side view) of FIG. 5A. FIGS. 5A and 5B are the top-face and side-face conceptual diagrams illustrating the intermediate charge portion 30c in the movement waiting position, which is the position of the intermediate charge portion 30c until the robot 20 moves the hot-melt adhesive 60 to the intermediate charge portion 30c. FIG. 5C is a side-face conceptual diagram illustrating the charging position of the intermediate charge portion 30c, at which the intermediate charge portion 30c charges the hot-melt adhesive 60 into the melting tank 30a after receiving it from the robot 20. As illustrated in FIGS. 5A to 5C, in an embodiment of the present disclosure, the intermediate charge portion 30c to receive the hot-melt adhesive 60 from the robot 20 and charge the received hot-melt adhesive 60 into the melting tank 30a is provided at a position adjacent to the melting tank 30a, and the robot 20 moves the hot-melt adhesive 60 to the melting tank 30a through the intermediate charge portion 30c.

Further, as described above, the melting tank lid portion 30b operates in conjunction with the intermediate charge portion 30c. Examples to implement such an interlocking operation include those using the intermediate charge portion 30c and/or a detection portion to detect the position of the intermediate charge portion 30c (in this case, the melting tank lid portion 30b is opened/closed based on the detection result).

The operations of the melting tank lid portion 30b and the intermediate charge portion 30c will be described below.

FIGS. 5A and 5B are diagrams in which the intermediate charge portion 30c has received the hot-melt adhesive 60 from the robot 20. As described above, the robot 20 holds the hot-melt adhesive 60 in the container 50 that is provided in the container installation portion 30d, and moves it to the intermediate charge portion 30c. That is, the aforementioned holding with the holding portion 20b of the robot 20 is released above on the container side in the direction of alignment of the table 30ct in the intermediate charge portion 30c in the movement waiting position. Then, the held hot-melt adhesive 60 drops downward, and the dropped hot-melt adhesive 60 is received on the container side in the direction of alignment of the table 30ct in the intermediate charge portion 30c.

Subsequently, as illustrated in FIG. 5C, the table 30ct of the intermediate charge portion 30c moves into the charging position. In conjunction therewith, the melting tank lid portion 30b opens the opening portion of the melting tank 30a. Then, in the charging position, the end on the tank side in the direction of alignment of the table 30ct is positioned above the opening portion of the melting tank 30a, as well as the table 30ct is inclined such that the tank side thereof in the direction of alignment becomes lower in the height direction than the container side thereof. That is, with the position and inclination of the table 30ct, the aforementioned hot-melt adhesive 60 received on the container side in the direction of alignment of the table 30ct is transported to the tank side in the direction of alignment, under the hot-melt adhesive 60's own weight and by the plurality of transport rolls 30cr. The hot-melt adhesive 60 transported to the end on the tank side in the direction of alignment of the table 30ct drops from this end, to be charged into the melting tank 30a from the opening portion of the melting tank 30a positioned below, in the height direction, the end of the table 30ct.

Thereafter, when the hot-melt adhesive 60 is charged into the melting tank 30a, the intermediate charge portion 30c moves to the movement waiting position. In conjunction therewith, the melting tank lid portion 30b closes the opening portion of the melting tank 30a. Then, the intermediate charge portion 30c waits for the next movement of the hot-melt adhesive 60 to be made by the robot 20, in the movement waiting position.

The container installation portion 30d is a substantially rectangular container stand on which the container 50 is mounted. The in-container detection portion CS is to detect the amount of the hot-melt adhesive 60 in the container 50. The in-container detection portion CS includes, for example, those using a scale that measures the weight of the container 50 and an imaging device that images the interior of the container 50.

### <<<AGV 40>>>

The AGV 40 is an automated guided vehicle to transport the new container 50 (the container 50 containing the hot-melt adhesive 60); replace the used container 50 (the container 50 having become empty of the hot-melt adhesive 60) provided in the tank container installation zone Z, with the new container 50; and transport the aforementioned used container 50.

FIGS. 1 and 2 illustrate the single AGV 40 as an example, but the number thereof may be two or more. Then, in an embodiment of the present disclosure, the AGV 40 transports the plurality of containers 50 containing the hot-melt adhesives 60 different from one another, and the plurality of containers 50 has the same specifications.

The AGV 40 includes: a table on which the container 50 is to be placed; a drive portion (not shown) for self-propelling; a load/unload portion (not shown) to load/unload the container 50 onto/from the table; an AGV position detection portion AP to detect the current position of the AGV 40; and an AGV table detection portion AT to detect whether the used container 50 and/or the new container 50 are placed on the table. Then, the AGV position detection portion AP includes, for example, those using a magnetic sensor or a laser sensor. Further, this AGV table detection portion AT includes, for example, those using a scale that measures the weight and an imaging device that images the top of the table. Further, in an embodiment of the present disclosure, two containers 50 can be placed on the table. That is, the AGV 40 is capable of moving to a container replacement position CP, which will be described later, of the hot-melt device 30 requiring replacement, while being loaded with the one new container 50 on the table, collecting the used container 50 with the aforementioned load/unload portion, and then providing the new container 50 (capable of replacement of the container 50).

The AGV 40 according to an embodiment of the present disclosure is capable of being self-propelled along the direction of alignment as a direction of movement. In other words, the absorbent article manufacturing apparatus 10 includes the transport member running lane GA along which the AGV 40 moves, and the transport member running lane GA is provided, along the direction of alignment, at a position adjacent in the lateral direction to the tank container installation zone Z (on the left side of the tank container installation zone Z). Further, the container replacement positions CP for replacing five types of the hot-melt adhesive 60 are provided in the transport member running lane GA. In FIGS. 1 and 2, the five container replacement positions CP (for convenience sake, referred to as a first container replacement position CP1, a second container replacement position CP2, a third container replacement position CP3, a fourth container replacement position CP4, a fifth container replacement position CP5, in the order from the back side to the front side) are provided corresponding to the five hot-melt devices 30. The AGV position detection portion AP detects the container replacement position CP, so that the AGV 40 can be self-propelled to positions at which the five types of hot-melt adhesive 60 are replaced. That is, in an embodiment of the present disclosure, the AGV 40 that is self-propelled to transport the container 50 is included, and the AGV 40 installs the transported container 50 onto the container installation portion 30d, and transports the used container 50 having been collected from the container installation portion 30d.

Further, the AGV 40 according to an embodiment of the present disclosure circulates on the outermost side of the absorbent article manufacturing apparatus 10. That is, in the absorbent article manufacturing apparatus 10, a circulation path along which the AGV 40 moves (not shown. the transport member running lane GA is a part of the circulation path) is provided so as to surround the processing section, a material feeding device, the robot 20, and the hot-melt devices 30, and forms a single loop line. That is, the AGV 40 moves along the circulation path, thereby circulating on the outermost side of the absorbent article manufacturing apparatus 10.

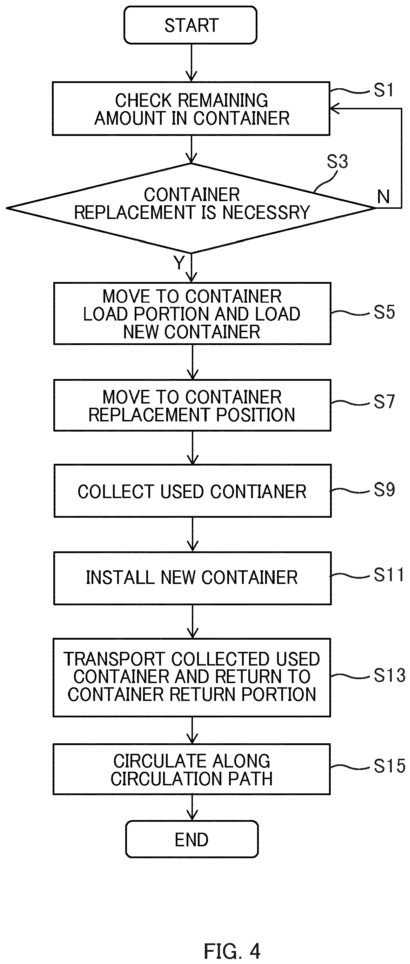

FIG. 4 is a flow chart illustrating operational control of the AGV 40. The operation of the AGV 40 according to an embodiment of the present disclosure will be described with reference to the flow chart. Here, for example, an operation of replacing the first hot-melt adhesive 61 with respect to the first hot-melt device 31 among the five hot-melt devices 30 will be described.

First, the amount of the first hot-melt adhesive 61 in the container 50 provided in the first container installation portion 31d is acquired by the first in-container detection portion 31CS (step S1). Then, when the amount of the first hot-melt adhesive 61 exceeds the threshold value (No in step S3), a state of waiting for the next acquisition of the first in-container detection portion 31CS is brought about. When the amount of the first hot-melt adhesive 61 is equal to or smaller than the threshold value, that is, when it is determined that replacement of the container is necessary (Yes in step S3), the next operation is performed.

That is, the AGV 40 acquires the current position using the AGV position detection portion AP, and acquires installation information of the container 50 with the AGV table detection portion AT. Then, the AGV 40 moves to a container load portion, with its table loaded with no container 50 (loaded with neither the used container 50 nor the new container 50), and is loaded with the new container 50 (step S5).

Subsequently, the AGV 40 loaded with the new container 50 moves to the first container replacement position CP1 of the first hot-melt device 31 requiring replacement (step S7). That is, the AGV 40 acquires a path to be self-propelled from a difference between the container load portion and the first container replacement position CP1, and moves along the path to a position at which the AGV position detection portion AP detects the first container replacement position CP1.

Subsequently, the AGV 40 collects the used container 50 from the first container installation portion 31d of the first hot-melt device 31 requiring replacement (step S9). That is, the AGV 40 uses the load/unload portion to collect the used container 50 placed in the container installation position of the first container installation portion 31d.

Subsequently, the AGV 40 installs the new container 50 placed on its table onto the first container installation portion 31d of the first hot-melt device 31 requiring replacement (step S11). That is, the AGV 40 uses the load/unload portion, to install the new container 50 placed on the table onto the first container installation portion 31d.

Subsequently, the AGV 40 loaded with the used container 50 moves to a container return portion (step S13). That is, the AGV 40 acquires a path to be self-propelled from a difference between the first container replacement position CP1 and the container return portion, and moves along the path to the container return portion. Then, the AGV 40 returns the used container 50 at the container return portion. Then, the AGV 40 circulates along the circulation path (step S15), and waits for the next operation.

### <<<Effectiveness of absorbent article manufacturing apparatus 10 according to embodiment of the present disclosure>>>

As described above, the absorbent article manufacturing apparatus 10 according to an embodiment of the present disclosure manufactures an absorbent article by bonding a material with the hot-melt adhesive 60, and includes the melting tank 30a to melt the hot-melt adhesive 60, the in-tank detection sensor TS to detect the amount of the hot-melt adhesive 60 in the melting tank 30a, and the robot 20 to move the hot-melt adhesive 60 in a solid state from the container 50 containing the hot-melt adhesive 60 to the melting tank 30a, based on the detection result of the in-tank detection sensor TS. Thus, as will be describe below, it becomes possible to restrain the hot-melt adhesive 60 from deteriorating in quality.

Conventionally, the melting tank 30a with the hot-melt adhesive 60 having been reduced by being used for joining is manually refilled. As a result, there is a case where the melting tank 30a is not refilled until immediately before running out of the hot-melt adhesive 60. In such a case, a worker charges a large amount of hot-melt adhesive 60 at a time into the melting tank 30a where the hot-melt adhesive 60 hardly exists. Then, when such a situation occurs, the hot-melt adhesive 60 deteriorates in quality.

That is, the melted hot-melt adhesive 60 in the melting tank 30a is controlled so as to be within a range set by the temperature detecting section and the heating section or be at a predetermined temperature, as described above. Under such control, when a large amount of hot-melt adhesive 60 in a solid state is charged at a time, excessive heating is performed by the heating section to melt the large amount of charged hot-melt adhesive 60. Then, when the excessive heating is performed, phenomena such as carbonization, viscosity increase, and gelation (hereinafter, these are referred to as adhesive state change, for convenience sake) occur in the hot-melt adhesive 60. Such an adhesive state change may have caused problems of malodor, discoloration, adhesion (viscosity) reduction in the hot-melt adhesive 60 (that is, such problems that the hot-melt adhesive 60 deteriorates in quality).

Further, the aforementioned adhesive state change may have caused filter clogging in the melting tank 30a, which may have required time and effort to clean the melting tank 30a. Further, due to such an adhesive state change, the hot-melt adhesive 60 may not have been stably applied to a material in the processing section, so that such problems may have arisen that application is not performed in a desired pattern or at a desired basis weight. Accordingly, problems related to the apparatus (device) may have been caused.

Further, as long as the solid hot-melt adhesive 60 is charged in a state where the melted hot-melt adhesive 60 sufficiently exists in the melting tank 30a, the solid hot-melt adhesive 60 is melted by the heat of the melted hot-melt adhesive 60 existing in the melting tank 30a. However, in the conventional example, the solid hot-melt adhesive 60 is charged in a state where the melted hot-melt adhesive 60 hardly exits in the melting tank 30a, so that the solid hot-melt adhesive 60 directly contacts the heating section and is heated. In this case also, the adhesive state change occurs in the hot-melt adhesive 60, and such a problem as deterioration in quality of the hot-melt adhesive 60 and/or problems related to the apparatus (device) may have caused.

Further, since the solid hot-melt adhesive 60 is charged at a time in a state where the melted hot-melt adhesive 60 hardly exists in the melting tank 30a, there is a possibility that the hot-melt adhesive 60 is supplied to the processing section before reaching a sufficient melted state in the melting tank 30a. This increases the possibility of deterioration in quality of the hot-melt adhesive 60 that is to be provided for adhesion.

On the other hand, in an embodiment of the present disclosure, the robot 20 moves the hot-melt adhesive 60 to the melting tank 30a based on the detection result of the in-tank detection sensor TS. Thus, such an event no longer occurs where the melting tank 30a is not refilled until immediately before running out of the hot-melt adhesive 60.

Accordingly, such events can be avoided, where the hot-melt adhesive 60 is excessively heated by the heating section; where the hot-melt adhesive 60 directly contacts the heating section and is heated; and where the hot-melt adhesive 60 is supplied to the processing section before reaching the sufficient melted state in the melting tank 30a. As a result, it is possible to restrain deterioration in quality of the hot-melt adhesive 60. Furthermore, it is also possible to restrain occurrence of the aforementioned problems related to the apparatus (device).

Further, in an embodiment of the present disclosure, as illustrated in FIGS. 1 and 2, the AGV 40 configured to be self-propelled to transport the container 50 is included, and the AGV 40 installs the transporting container 50 in the container installation position, and transports the used container 50 collected from the container installation position.

That is, the AGV 40 is capable of loading the new container 50 and being self-propelled to move (transports) it; automatically collecting the used container 50; automatically installing the loaded new container 50 in the container installation position; and being self-propelled to move (transport) the used container 50 that have been collected and loaded, to return it to the container return portion. It is possible to perform work of replacing the container 50 more swiftly, as compared with the case where such work is manually performed.

Further, in an embodiment of the present disclosure, as illustrated in FIGS. 1 and 2, the self-propelled robot lane GR along which the robot 20 is self-propelled and the transport member running lane GA along which the AGV 40 is self-propelled are included, and the self-propelled robot lane GR and the transport member running lane GA are located on both the sides, in a direction (lateral direction) orthogonal to the direction of alignment, of the tank container installation zone Z in which the plurality of melting tanks 30a and containers 50 are aligned in the direction of alignment, such that the self-propelled robot lane GR and the transport member running lane GA are both along the direction of alignment.

That is, as illustrated in FIGS. 1 and 2, the self-propelled robot lane GR, the tank container installation zone Z, and the transport member running lane GA are provided along the direction of alignment, and the self-propelled robot lane GR, the tank container installation zone Z, and the transport member running lane GA are laid out in this order in the lateral direction orthogonal to the direction of alignment.

As a result, the robot 20 and the AGV 40 can implement efficient movements. That is, if either the self-propelled robot lane GR or the transport member running lane GA is not on the side adjacent in the lateral direction to the tank container installation zone Z, as well as is not provided along the direction of alignment, the robot 20 and the AGV 40 less efficiently moves as compared with the case where the self-propelled robot lane GR and the transport member running lane GA are located on both sides adjacent in the lateral direction to the tank container installation zone Z, as well as are provided along the direction of alignment.

Further, in an embodiment of the present disclosure, the AGV 40 transports the plurality of containers 50 containing the hot-melt adhesives 60 different from each other, and the plurality of containers 50 has the same specifications.

If the specifications (size, type, etc., (e.g., so-called folding container, flexible container bag, cardboard container, etc.)) of the containers 50 vary with the types of the hot-melt adhesive 60, it is difficult to support the containers 50 having different specifications with a single type of the AGV 40, so that a plurality of types of AGVs 40 may have to be prepared corresponding to the specifications of the containers 50. In contrast, in an embodiment of the present disclosure, since the plurality of containers 50 has the same specifications, it is unnecessary to prepare the plurality of AGVs 40 corresponding to the specifications of the containers 50, so that the costs related to the AGV 40 can be reduced.

Further, in an embodiment of the present disclosure, included is the residual detection portion HS configured to detect that no residual hot-melt adhesive 60 adheres to the robot 20, when the robot 20 completes movement of the hot-melt adhesive 60.

Accordingly, when the hot-melt adhesive 60 different, in type, from the residual hot-melt adhesive 60 is held by the robot 20 in a state where the residual hot-melt adhesive 60 remains in the robot 20, both types of the hot-melt adhesive 60 are restrained from being mixed with each other.

Further, in an embodiment of the present disclosure, included is the intermediate charge portion 30c configured to receive the hot-melt adhesive 60 from the robot 20 and charge the received hot-melt adhesive 60 to the melting tank 30a, the intermediate charge portion 30c being provided in a position adjacent to the melting tank 30a, wherein the robot 20 moves the hot-melt adhesive 60 to the melting tank 30a via the intermediate charge portion 30c.

Accordingly, it is possible to prevent adverse effects of oil smoke (lampblack) (those in a smoke state being generated when the hot-melt adhesive 60 is heated and melted) . The oil smoke is generated when the hot-melt adhesive 60 is heated, and thus is generated in the melting tank 30a where heating is performed. Then, the oil smoke in the melting tank 30a is discharged above the melting tank 30a when the opening portion is opened. That is, with the provision of the intermediate charge portion 30c, the robot 20 does not have to move above the opening portion of the melting tank 30a, and thus the adverse effects of the oil smoke onto the robot 20 can be prevented.

Here, the adverse effects of the oil smoke indicates, for example, that when the oil smoke, generally having a high temperature and a black soot, is applied to the robot 20, for example, the heat of the oil smoke melts the hot-melt adhesive 60 held by the robot 20, to increase an adhesive force of the hot-melt adhesive 60, so that the hot-melt adhesive 60 is not easily detached from the robot 20, thereby remaining at the holding portion 20b. Another adverse effect is that the residual detection portion HS of the robot 20 is covered with black soot of the oil smoke, so that the residual detection portion HS cannot detect the residual of the hot-melt adhesive 60 of the holding portion 20b. Still another adverse effect is that the oil smoke adheres to the robot 20, thereby contaminating the robot 20.

Further, in an embodiment of the present disclosure, the melting tank 30a includes the openable/closable melting tank lid portion 30b, and when the intermediate charge portion 30c charges the hot-melt adhesive 60 to the melting tank 30a, the melting tank lid portion 30b is opened.

Thus, a period of time during which the opening portion of the melting tank 30a is opened is minimized, so that the oil smoke can be restrained from being discharged from the opening portion.

### ===Other embodiments===

The above embodiment of the present disclosure is simply for facilitating the understanding of the present disclosure and is not in any way to be construed as limiting the present disclosure. The present disclosure may variously be changed or altered without departing from the scope of the appended claims.

An embodiment of the present disclosure provides an example where five types of the hot-melt adhesive 60 are used, but it is not limited thereto. For example, the hot-melt adhesive 60 may have four types or less, or six types or more.

Further, in an embodiment of the present disclosure, the AGV 40 waits for the next operation while circulating along the circulation path, but it is not limited thereto. For example, the AGV 40 may move to its home position and wait for the next operation.

Further, in an embodiment of the present disclosure described above, an example is given in which the AGV 40 is loaded with and transports two containers 50, but it is not limited thereto. For example, the AGV 40 may be loaded with and transport one container 50, or three or more containers 50.

Further, in an embodiment of the present disclosure described above, such an example is given that the holding portion 20b of the robot 20 is capable of holding the five types of the hot-melt adhesive 60, it is not limited thereto. For example, the holding portion 20b may be replaced corresponding to the types of the hot-melt adhesive 60, to hold five types of the hot-melt adhesive 60.

Further, in an embodiment of the present disclosure described above, such an example is given that the container load portion and the container return portion are provided, it is not limited thereto. For example, a container load/return portion or the like may be provided, which performs loading and returning of the container.

Further, in an embodiment of the present disclosure described above (referred to as a first embodiment), the intermediate charge portion 30c in the hot-melt device 30 is of a type of moving (shifting) between the movement waiting position (FIG. 5B) of a horizontal state and the charging position (FIG. 5C) of an inclined state, but it is not limited thereto. For example, the intermediate charge portion 30c may be always inclined without state shifting.

FIG. 5D is a side-face conceptual diagram illustrating the intermediate charge portion 30c according to a second embodiment. The intermediate charge portion 30c according to the second embodiment has a flat shape on the upper side in the height direction, and includes the substantially rectangular table 30ct having a length in the direction of alignment to receive the hot-melt adhesive 60 from the robot 20 and transport it above the opening portion of the melting tank 30a. Then, as illustrated in FIG. 5D, the table 30ct includes the plurality of transport rolls 30cr to transport the received hot-melt adhesive 60 in the direction of alignment of the table 30ct. The plurality of transport rolls 30cr form an upper face of the table 30ct (i.e., are positioned at the upper end in the height direction of the table 30ct as well as are provided from one end to the other end in the direction of alignment), and rotate about the rotation shafts along the lateral direction (direction penetrating the paper in FIGS. 5B and 5C).

The intermediate charge portion 30c according to the second embodiment receives the hot-melt adhesive 60 dropping from the aforementioned holding portion 20b on the container side in the direction of alignment of the table 30ct. On the other hand, the end on the tank side in the direction of alignment of the table 30ct is positioned above the opening portion of the melting tank 30a. Then, the table 30ct of the intermediate charge portion 30c is inclined such that the tank side in the direction of alignment is positioned below, in the height direction, the container side. That is, by virtue of the position and inclination of the table 30ct, the aforementioned hot-melt adhesive 60 having been received on the container side in the direction of alignment of the table 30ct is transported to the tank side in the direction of alignment with the plurality of transport rolls 30cr under the own weight of the hot-melt adhesive 60. The hot-melt adhesive 60 transported to the end on the tank side in the direction of alignment of the table 30ct drops from the end, to be charged into the melting tank 30a from the opening portion of the melting tank 30a positioned below, in the height direction, the end.

Further, in an embodiment of the present disclosure described above, the robot 20 in common moves the hot-melt adhesive 60 to each of the plurality of melting tanks 30a, but it is not limited thereto. For example, the robots 20 may be respectively dedicated to the plurality of melting tanks 30a (e.g., the same number of robots 20 as the number of the melting tanks 30a may be prepared).

However, the above-described embodiment is preferable in that the number of the robots 20 can be reduced.

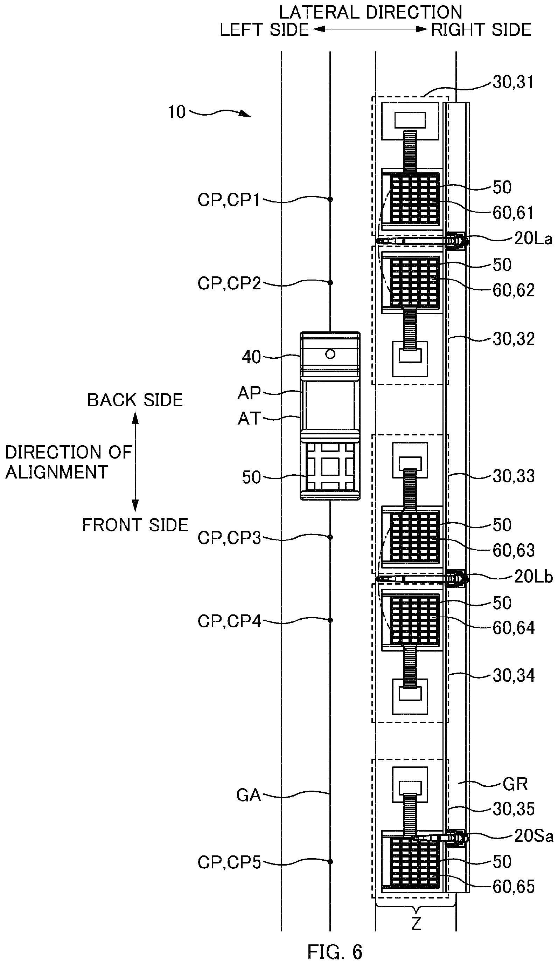

Further, in an embodiment of the present disclosure described above, when the robot 20 is self-propelled to reach the corresponding position RP corresponding to one melting tank 30a among the plurality of melting tanks 30a, the hot-melt adhesive 60 is transported to the one melting tank 30a, but it is not limited thereto. For example, the robot 20 does not have to be self-propelled, as illustrated in FIG. 6.

FIG. 6 is a top-face conceptual diagram illustrating the absorbent article manufacturing apparatus 10 according to a third embodiment when viewed from above. As illustrated in FIG. 6, a point different from the first embodiment (FIGS. 1 and 2) is that a robot 20La, a robot 20Lb, and a robot 20Sa are not self-propelled (but each staying at a given place). In a third embodiment, the robot 20La and the robot 20Lb each are in charge of two of the hot-melt devices 30. That is, the robot 20La is in charge of the first hot-melt device 31 and the second hot-melt device 32, while the robot 20Lb is in charge of the third hot-melt device 33 and the fourth hot-melt device 34. Then, the robot 20Sa is in charge of the single hot-melt device 30. That is, the robot 20Sa is in charge of the fifth hot-melt device 35.

However, the aforementioned embodiment in which the robot 20 is given as a self-propelled robot is more preferable in that the number of robots can be further reduced.

## Claims

1. An apparatus (10) for manufacturing an absorbent article by bonding a material with a hot-melt adhesive (60), the apparatus comprising:
a melting tank (30a) configured to melt the hot-melt adhesive; **characterized in that** the apparatus comprises:
a detection sensor (TS) configured to detect an amount of the hot-melt adhesive in the melting tank (30a); and
an articulated robot (20) which is a device configured to hold the hot-melt adhesive (60) in a container (50) containing the hot-melt adhesive and to move the hot-melt adhesive from the container containing the hot-melt adhesive to the melting tank (30a) based on a detection result of the detection sensor (TS).

2. An apparatus for manufacturing an absorbent article according to claim 1, further comprising:
a plurality of melting tanks (30), wherein
the robot (20) in common is configured to move the hot-melt adhesive (60) to each of the plurality of melting tanks.

3. An apparatus for manufacturing an absorbent article according to claim 2, wherein
the robot (20) is a self-propelled robot, and
when the robot is self-propelled to reach a corresponding position, the corresponding position corresponding to one melting tank (30a) among the plurality of melting tanks (30), the robot moves the hot-melt adhesive (60) to the one melting tank.

4. An apparatus for manufacturing an absorbent article according to any one of claims 1 to 3, further comprising:
a container transport member (40) configured to be self-propelled to transport the container (50), wherein
the container transport member (40) is configured to install the transporting container in the container installation position, and to transport the used container collected from the container installation position.

5. An apparatus for manufacturing an absorbent article according to claim 4 further comprising:
a self-propelled robot lane (GR) along which the robot (20) is self-propelled;
a transport member running lane (GA) along which the container transport member (40) is self-propelled; and
a tank container installation zone (Z) in which the plurality of melting tanks (30) and containers (50) are aligned in the direction of alignment, wherein
the self-propelled robot lane (GR) and the transport member running lane (GA) are located on both sides, in a direction orthogonal to a direction of alignment, of the tank container installation zone (z), and
the self-propelled robot lane and the transport member running lane are both along the direction of alignment.

6. An apparatus for manufacturing an absorbent article according to claim 4 or 5, wherein
the container transport member (40) is configured to transport a plurality of containers (50) containing hot-melt adhesives (60) different from one another, and
the plurality of containers has same specifications.

7. An apparatus for manufacturing an absorbent article according to any one of claims 1 to 6, further comprising:
a residual detecting portion (HS) configured to detect whether no residual hot-melt adhesive (60) adheres to the robot (20), when the robot completes movement of the hot-melt adhesive.

8. An apparatus for manufacturing an absorbent article according to any one of claims 1 to 7, further comprising:
an intermediate charge portion (30c) configured to receive the hot-melt adhesive (60) from the robot (20), and to charge the received hot-melt adhesive to the melting tank (30a), the intermediate charge portion being provided adjacent to the melting tank, wherein
the robot (20) is configured to move the hot-melt adhesive (60) to the melting tank (30a) via the intermediate charge portion (30c).

9. An apparatus for manufacturing an absorbent article according to claim 8, wherein
the melting tank (30a) includes an openable/closable lid portion (30b), and
when the intermediate charge portion (30c) charges the hot-melt adhesive (60) into the melting tank, the lid portion is opened.

10. A method for manufacturing an absorbent article by bonding a material with a hot-melt adhesive (60), **characterized in that** the method comprises:
detecting, using a detection sensor (TS), an amount of the hot-melt adhesive (60) in a melting tank (30a), the melting tank configured to melt the hot-melt adhesive;
holding the hot-melt adhesive (60), using an articulated robot (20), in a container (50) containing the hot-melt adhesive; and
moving the hot-melt adhesive, using the articulated robot(20), from the container (50) containing the hot-melt adhesive to the melting tank (30a) based on a detection result of the detection sensor (TS).

## Patentansprüche

1. Vorrichtung (10) zur Herstellung eines absorbierenden Artikels durch Verbinden eines Materials mit einem Heißschmelzkleber (60), wobei die Vorrichtung Folgendes umfasst:
einen Schmelztank (30a), der zum Schmelzen des Heißschmelzklebers konfiguriert ist; **dadurch gekennzeichnet, dass** die Vorrichtung Folgendes umfasst:
einen Erkennungssensor (TS), der zur Erkennung einer Menge des Heißschmelzklebers in dem Schmelztank (30a) konfiguriert ist; und
einen Knickarmroboter (20), der eine Einrichtung ist, die zum Halten des Heißschmelzklebers (60) in einem Behälter (50), der den Heißschmelzkleber enthält, und zum Bewegen des Heißschmelzklebers aus dem Behälter, der den Heißschmelzkleber enthält, zu dem Schmelztank (30a), basierend auf einem Erkennungsergebnis des Erkennungssensors (TS), konfiguriert ist.

2. Vorrichtung zur Herstellung eines absorbierenden Artikels nach Anspruch 1, die weiter Folgendes umfasst:
eine Vielzahl von Schmelztanks (30), wobei
der gemeinsame Roboter (20) zur Bewegung des Heißschmelzklebers (60) zu jedem der Vielzahl von Schmelztanks konfiguriert ist.

3. Vorrichtung zur Herstellung eines absorbierenden Artikels nach Anspruch 2, wobei
der Roboter (20) ein selbstfahrender Roboter ist und,
wenn der Roboter selbst fährt, um eine entsprechende Position zu erreichen, wobei die entsprechende Position einem Schmelztank (30a) unter der Vielzahl von Schmelztanks (30) entspricht, der Roboter den Heißschmelzkleber (60) zu dem einen Schmelztank bewegt.

4. Vorrichtung zur Herstellung eines absorbierenden Artikels nach einem der Ansprüche 1 bis 3, die weiter Folgendes umfasst:
ein Behältertransportelement (40), das zum Selbstfahren konfiguriert ist, um den Behälter (50) zu transportieren, wobei
das Behältertransportelement (40) zum Einsetzen des transportierenden Behälters in die Behältereinsetzungsposition und zum Transport des verwendeten Behälters, der von der Behältereinsetzungsposition abgeholt wurde, konfiguriert ist.

5. Vorrichtung zur Herstellung eines absorbierenden Artikels nach Anspruch 4, die weiter Folgendes umfasst:
eine Fahrspur für den selbstfahrenden Roboter (GR), entlang der der Roboter (20) selbst fährt;
eine Laufspur für das Transportelement (GA), entlang der das Behältertransportelement (40) selbst fährt; und
eine Tankbehältereinsetzungszone (Z), in der die Vielzahl von Schmelztanks (30) und Behältern (50) in der Richtung der Ausrichtung ausgerichtet werden,
wobei
sich die Fahrspur für den selbstfahrenden Roboter (GR) und die Laufspur für das Transportelement (GA) auf beiden Seiten, in einer Richtung senkrecht zu einer Richtung der Ausrichtung, der Tankbehältereinsetzungszone (Z) befinden und
die Fahrspur für den selbstfahrenden Roboter und die Laufspur für das Transportelement beide entlang der Richtung der Ausrichtung verlaufen.

6. Vorrichtung zur Herstellung eines absorbierenden Artikels nach Anspruch 4 oder 5, wobei
das Behältertransportelement (40) zum Transport einer Vielzahl von Behältern (50), die Heißschmelzkleber (60) enthalten, die voneinander verschieden sind, konfiguriert ist und
die Vielzahl von Behältern gleiche Spezifikationen aufweist.

7. Vorrichtung zur Herstellung eines absorbierenden Artikels nach einem der Ansprüche 1 bis 6, die weiter Folgendes umfasst:
einen Rückstandserkennungsteil (HS), der zur Erkennung konfiguriert ist, ob kein rückständiger Heißschmelzkleber (60) an dem Roboter (20) haftet, wenn der Roboter die Bewegung des Heißschmelzklebers beendet.

8. Vorrichtung zur Herstellung eines absorbierenden Artikels nach einem der Ansprüche 1 bis 7, die weiter Folgendes umfasst:
einen Zwischenbeladungsteil (30c), der zur Aufnahme des Heißschmelzklebers (60) von dem Roboter (20) und zur Beladung des aufgenommenen Heißschmelzklebers in den Schmelztank (30a), wobei der Zwischenbeladungsteil benachbart zum Schmelztank bereitgestellt ist, wobei
der Roboter (20) zur Bewegung des Heißschmelzklebers (60) zu dem Schmelztank (30a) über den Zwischenbeladungsteil (30c) konfiguriert ist.

9. Vorrichtung zur Herstellung eines absorbierenden Artikels nach Anspruch 8, wobei
der Schmelztank (30a) einen öffenbaren/schließbaren Deckelteil (30b) einschließt und,
wenn der Zwischenbeladungsteil (30c) den Heißschmelzkleber (60) in den Schmelztank lädt, der Deckelteil geöffnet ist.

10. Verfahren zur Herstellung eines absorbierenden Artikels durch Verbinden eines Materials mit einem Heißschmelzkleber (60), **dadurch gekennzeichnet, dass** das Verfahren Folgendes umfasst:
Erkennen, unter Verwendung eines Erkennungssensors (TS), einer Menge des Heißschmelzklebers (60) in einem Schmelztank (30a), wobei der Schmelztank zum Schmelzen des Heißschmelzklebers konfiguriert ist;
Halten des Heißschmelzklebers (60), unter Verwendung eines Knickarmroboters (20), in einem Behälter (50), der den Heißschmelzkleber enthält, und
Bewegen des Heißschmelzklebers, unter Verwendung des Knickarmroboters (20), aus dem Behälter (50), der den Heißschmelzkleber enthält, zu dem Schmelztank (30a), basierend auf einem Erkennungsergebnis des Erkennungssensors (TS).

## Revendications

1. Appareil (10) de fabrication d'un article absorbant par la liaison d'un tissu au moyen d'un adhésif thermofusible (60), l'appareil comportant :
un cuve de fusion (30a) configurée pour faire fondre l'adhésif thermofusible ; **caractérisé en ce que** l'appareil comporte :
un capteur de détection (TS) configuré pour détecter une quantité de l'adhésif thermofusible dans la cuve de fusion (30a) ; et
un robot articulé (20) qui est un dispositif configuré pour contenir l'adhésif thermofusible (60) dans un contenant (50) qui contient l'adhésif thermofusible et pour déplacer l'adhésif thermofusible en provenance du contenant qui contient l'adhésif thermofusible jusque dans la cuve de fusion (30a) en se basant sur un résultat de détection du capteur de détection (TS).

2. Appareil de fabrication d'un article absorbant selon la revendication 1, comportant par ailleurs :
une pluralité de cuves de fusion (30), dans lequel le robot (20) en commun est configuré pour déplacer l'adhésif thermofusible (60) jusque dans chacune de la pluralité de cuves de fusion.

3. Appareil de fabrication d'un article absorbant selon la revendication 2, dans lequel
le robot (20) est un robot autopropulsé, et
quand le robot est autopropulsé pour atteindre une position correspondante, la position correspondante correspondant à une cuve de fusion (30a) parmi la pluralité de cuves de fusion (30), le robot déplace l'adhésif thermofusible (60) jusque dans ladite une cuve de fusion.

4. Appareil de fabrication d'un article absorbant selon l'une quelconque des revendications 1 à 3, comportant par ailleurs :
un élément de transport de contenant (40) configuré pour être autopropulsé à des fins de transport du contenant(50), dans lequel
l'élément de transport de contenant (40) est configuré pour installer le contenant de transport dans la position d'installation de contenant, et pour transporter le contenant ayant servi collecté en provenance de la position d'installation de contenant.

5. Appareil de fabrication d'un article absorbant selon la revendication 4, comportant par ailleurs :
une voie pour robot autopropulsé (GR) le long de laquelle le robot (20) est autopropulsé ;
une voie d'avance pour élément de transport (GA) le long de laquelle l'élément de transport de contenant (40) est autopropulsé ; et
une zone d'installation de contenant de cuve (Z) dans laquelle la pluralité de cuves de fusion (30) et les contenants (50) sont alignés dans la direction de l'alignement, dans lequel
la voie pour robot autopropulsé (GR) et la voie d'avance pour élément de transport (GA) sont situées des deux côtés, dans une direction orthogonale par rapport à une direction de l'alignement, de la zone d'installation de contenant de cuve (Z), et
la voie pour robot autopropulsé et la voie d'avance pour élément de transport sont toutes les deux dans la direction de l'alignement.

6. Appareil de fabrication d'un article absorbant selon la revendication 4 ou la revendication 5, dans lequel
l'élément de transport de contenant (40) est configuré pour transporter une pluralité de contenants (50) qui contiennent des adhésifs thermofusibles (60) différents les uns des autres, et
les contenants de la pluralité de contenants ont les mêmes spécifications.

7. Appareil de fabrication d'un article absorbant selon l'une quelconque des revendications 1 à 6, comportant par ailleurs :
une partie de détection de résidu (HS) configurée pour détecter qu'aucun adhésif thermofusible résiduaire (60) n'a adhéré sur le robot (20), quand le robot a effectué le déplacement de l'adhésif thermofusible.

8. Appareil de fabrication d'un article absorbant selon l'une quelconque des revendications 1 à 7, comportant par ailleurs :
une partie de charge intermédiaire (30c) configurée pour recevoir l'adhésif thermofusible (60) en provenance du robot (20), et pour charger l'adhésif thermofusible reçu jusque dans la cuve de fusion (30a), la partie de charge intermédiaire étant mise en œuvre de manière adjacente par rapport à la cuve de fusion, dans lequel
le robot (20) est configuré pour déplacer l'adhésif thermofusible (60) jusque dans la cuve de fusion (30a) par le biais de la partie de charge intermédiaire (30c).

9. Appareil de fabrication d'un article absorbant selon la revendication 8, dans lequel
la cuve de fusion (30a) comprend une partie formant couvercle en mesure d'être ouvert/fermé (30b), et
quand la partie de charge intermédiaire (30c) charge l'adhésif thermofusible (60) dans la cuve de fusion, la partie formant couvercle est ouverte.

10. Procédé de fabrication d'un article absorbant par la liaison d'un tissu au moyen d'un adhésif thermofusible (60), **caractérisé en ce que** le procédé comporte les étapes consistant à :
détecter, au moyen d'un capteur de détection (TS), une quantité de l'adhésif thermofusible (60) dans une cuve de fusion (30a), la cuve de fusion étant configurée pour faire fondre l'adhésif thermofusible ;
contenir l'adhésif thermofusible (60), au moyen d'un robot articulé (20), dans un contenant (50) qui contient l'adhésif thermofusible ; et
déplacer l'adhésif thermofusible, au moyen du robot articulé (20), en provenance du contenant (50) qui contient l'adhésif thermofusible jusque dans la cuve de fusion (30a) en se basant sur un résultat de détection du capteur de détection (TS) .
